(19) Europäisches Patentamt
European Patent Office
Office européen des brevets

(11) Numéro de publication: **0 365 383 A1**

(12) **DEMANDE DE BREVET EUROPEEN**

(21) Numéro de dépôt: **89402644.2**

(22) Date de dépôt: **27.09.89**

(51) Int. Cl.5: **C07C 51/10 , C07C 63/06 , C07C 65/21 , C07C 63/70**

(30) Priorité: **05.10.88 FR 8813008**

(43) Date de publication de la demande: **25.04.90 Bulletin 90/17**

(84) Etats contractants désignés: **AT BE CH DE ES FR GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Huser, Marc**
**36, rue du Fossé Rietberg**
**F-67100 Strasbourg(FR)**
Inventeur: **Metz, François**
**22, rue du Condé**
**F-69009 Lyon(FR)**
Inventeur: **Osborn, John**
**10, rue Daniel Hirtz**
**F-67000 Strasbourg(FR)**

(74) Mandataire: **Vignally, Noel et al**
**Rhône-Poulenc Chimie Service Brevets**
**Chimie 25 Quai Paul Doumer**
**F-92408 Courbevoie Cédex(FR)**

(54) **Procédé d'hydroxycarbonylation.**

(57) La présente invention concerne un procédé d'hydroxycarbonylation mettant en oeuvre un nouveau catalyseur constitué d'un complexe palladium-phosphine, celle-ci présentant un pKa supérieur ou égal à 7, un dérivé aromatique chloré, du monoxyde de carbone et de l'eau.

EP 0 365 383 A1

## PROCEDE D'HYDROXYCARBONYLATION

La présente invention concerne un procédé d'hydroxycarbonylation. Elle concerne plus particulièrement l'hydroxycarbonylation de dérivés aromatiques chlorés en présence d'un catalyseur à base de palladium.

La présente invention a pour objet un procédé d'hydroxycarbonylation en milieu homogène de dérivés aromatiques chlorés en présence d'un catalyseur à base de palladium.

Le catalyseur à base de palladium est précisément choisi parmi les complexes du palladium et d'une phosphine. Cette phosphine doit présenter un pKa supérieur à 7, tel que défini par WmA HENDERSON, Jr ; C.A. STREULI dans le journal of American Chemical Society, 82, 1960, 5791.

On peut citer parmi cette classe de phosphines non limitativement :

- la tricyclohexyl phosphine,
- la triisopropyl phosphine,
- la triéthyl phosphine,
- la tri n-butyl phosphine,
- la tritertiobutyl phosphine,
- la dicyclohexylphényl phosphine.

Parmi cette class de phosphines présentant un pKa supérieur à 7, on préfère utiliser les phosphines qui présentent un angle de cône tel que défini par C.A. TOLMAN, dans le Journal of American Chemical Socieity, 92, 1970, 2956 compris entre 160 et 180°.

Font partie de ce domaine préférentiel les phosphines suivantes

- la tricyclohexyl phosphine
- la triisopropyl phosphine
- la dicyclohexyl phényl phosphine

On préfère tout particulièrement utiliser la tricyclohexylphosphine.

Le complexe de la présente invention répond à la formule (I) suivante :

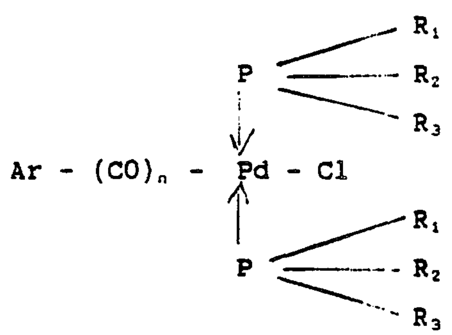

(I)

dans laquelle :

- $R_1$, $R_2$, $R_3$ représentent chacun des groupes identiques ou différents choisis parmi les radicaux cyclohexyle, isopropyle, l'un des groupes $R_1$, $R_2$ ou $R_3$ pouvant être remplacé par un groupe phényle lorsque les deux autres représentent un groupe cyclohexyle,
- Ar représente un radical aromatique mono, polycyclique ou hétérocyclique.
- n est égal à 0 ou 1.

Le complexe de formule (I) précédemment décrit est notamment utilisé pour catalyser les réactions d'hydroxycarbonylation selon le mécanisme réactionnel suivant :

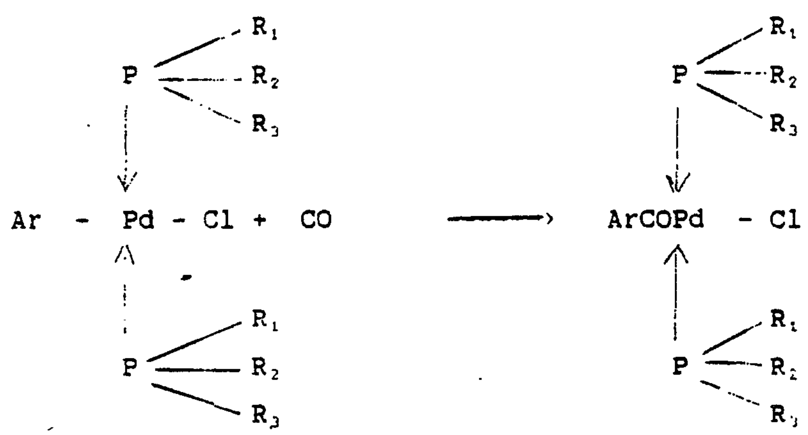

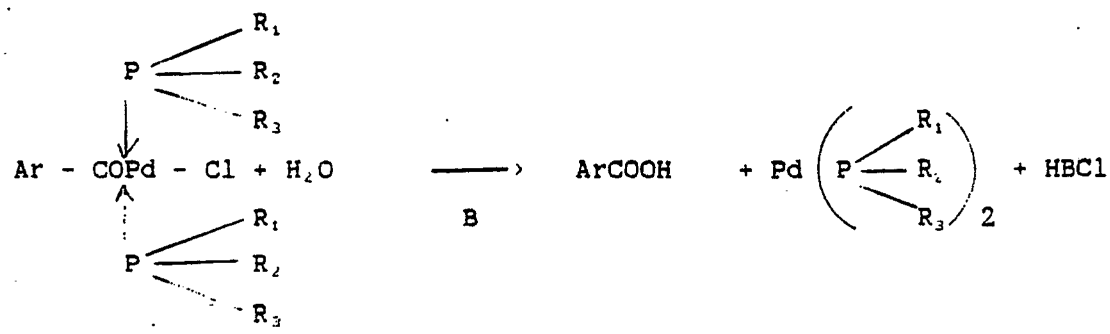


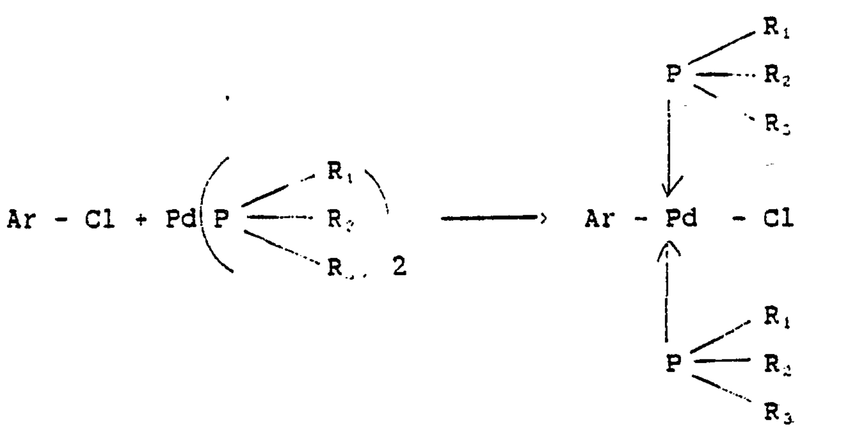

qui d'une manière simplifiée peut être résumée par l'équation suivante :

$$H_2O + ArCl + CO \xrightarrow[B]{\text{catalyseur à base de Pd}} ArCOOH + Cl\,B\,H$$

Dans les équations précédentes, le terme "R" signifie soit un groupe cyclohexyle, soit un groupe phényle, soit un groupe isopropyle. Le phosphore peut être ligandé à 3 groupes équivalents comme dans la tricyclohexyl phosphine ou par des groupes différents comme dans la dicyclohexylphényl phosphine.

Le dérivé aromatique chloré (ArCl) peut être mono, polycyclique ou hétérocyclique. Il peut être éventuellement substitué par un groupe alkoxy, alkyle, alkylcarbonyle, cycloalkyle, cycloalkoxy, halogéno,

halogénoalkyle, halogénoalkoxy, aryle, aryloxy, halogénoaryle, halogénoaryloxy, alkylaryle, aralkyle, alkylcarbonyloxy, cycloalkylcarbonyloxy.

Les chaines alkyles des divers groupes alkyle ou alkoxy contiennent de préférence 1 à 6 atomes de carbone, les groupes aryle contiennent de préférence 6 à 18 atomes de carbone.

On préfère utiliser les dérivés aromatiques monocycliques non substitués ou substitués par un groupe alkoxy, contenant 1 à 6 atomes de carbone, alkyle contenant 1 à 6 atomes de carbone, chloro, fluoro, alkyl carbonyle dont la chaîne alkyle contient 1 à 6 atomes de carbone.

Parmi les dérivés aromatiques chlorés utilisables dans le procédé de l'invention, on peut citer à titre illustratif :

- le chlorobenzène
- les dichlorobenzènes
- les chlorofluorobenzènes
- les chlorotoluènes
- les chloroanisoles
- les chloronaphtalènes
- les chlorobenzoates de méthyle, éthyle, propyle,
- la méthylchlorophénylcétone
- les chlorobiphényles
- le chloroindole
- le chlorothiophène

Une base (B) est nécessaire pour neutraliser l'acide chlorhydrique formé au cours de la réaction d'hydroxycarbonylation. Cette base peut être constituée de la phosphine elle-même ou d'une base différente. Si cette base est différente de la phosphine, elle a de préférence un pKa supérieur à celui de la phosphine de manière à ce que cette dernière ne joue pas inutilement le rôle de base neutralisante.

Elle doit de préférence être soluble dans le milieu réactionnel. Comme le milieu réactionnel est biphasique et constitué d'eau et d'un solvant organique, les bases minérales ainsi que les bases organiques peuvent être utilisées. Parmi lés bases organiques, on préfère utiliser les trialkylamines et par exemple la triéthylamine, la triisopropylamine, la tri-n butylamine. Les bases minérales peuvent aussi être utilisées telles que le carbonate de sodium ou l'hydroxyde de sodium.

Le solvant utilisé pour la mise en oeuvre de l'invention est choisi parmi
les solvants aromatiques hydrocarbonés tels que :
- le toluène
- les xylènes

les éthers, tels que :
- le dioxane

les cétones, telles que :
- la méthylisobutylcétone

les nitriles, tels que :
- le benzonitrile

les amides, tels que :
- le diméthylformamide

Les réactifs tels que le dérivé chloro aromatique ou la base peuvent servir de milieu réactionnel.

Le complexe de formule I peut être utilisé tel quel, comme catalyseur.

Le complexe de formule I peut être aussi forme "in situ" par au moins trois méthodes de mise en oeuvre.

Selon une première méthode de mise en oeuvre du procédé de l'invention, on met en contact un composé de formule (II) suivante :

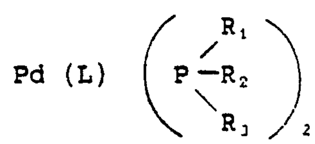

( II )

dans laquelle :
- le motif L représente un groupe labile en présence de ArCl.
- les groupes $R_1$, $R_2$, $R_3$ ont la même signification que dans la formule (I) avec un dérivé halogéno aromatique de formule ArCl du monoxyde de carbone et l'eau dans un solvant.

Selon une deuxième méthode de mise en oeuvre du procédé de l'invention on met en présence un complexe de palladium à l'état d'oxydation zéro tel que :

Pd $(L)_3$, au moins deux équivalents de phosphine répondant à la formule :

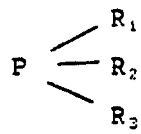

avec le dérivé chloroaromatique de formule ArCl, du monoxyde de carbone et l'eau.

Selon une troisième méthode de mise en oeuvre du procédé de l'invention, on met en présence un sel de palladium à l'état d'oxydation II choisi par exemple parmi le dichlorure, le dibromure, le diiodure de palladium, le diacétate de palladium, le nitrate de palladium, le sulfate de palladium, l'oxyde de palladium avec le dérivé chloroaromatique, du monoxyde de carbone, l'eau et au moins deux équivalents de phosphine de formule :

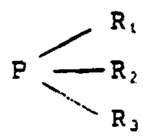

Au sens de la présente invention, on entend par groupe labile (L) tout groupe qui en présence de ArCl peut être facilement échangeable.

Parmi ces groupes, on peut citer non limitativement :

- la dibenzylidène acétone (DBA)
- les groupes alkylène et de préférence l'éthylène

Lorsque l'on part d'un complexe du palladium ne contenant pas de phosphine (deuxième ou troisième méthode de mise en oeuvre), on préfère utiliser au moins 2 mol de phosphine par atome gramme de palladium et de préférence entre 2 et 10000 mol et tout particulièrement entre 2 et 5.

On préfère utiliser une quantité de solvant telle que la concentration en complexe ou en sel de palladium dans le milieu soit comprise entre $10^{-5}$ et 100 mmol par litre.

La concentration minimale en base doit correspondre à la stoéchiométrie de la réaction. Elle peut être utilisée en quantité nettement supérieure et même être utilisée comme solvant. Il faut qu'en fin de réaction, on n'ait pas épuisé la base.

La concentration du dérivé aromatique chloré peut varier dans de larges limites puisqu'il peut être utilisé comme solvant. Il est dans ce cas facilement recyclé.

La concentration de l'eau peut aussi varier dans de larges limites puisqu'elle peut être utilisée comme solvant. Lorsqu'elle n'est pas utilisée comme solvant, un rapport molaire de 1 à 5 par rapport au dérivé chloroaromatique est tout à fait conseillé.

La température réactionnelle est avantageusement comprise entre 50 et 250° C et de préférence entre 100 et 200° C.

La pression partielle de monoxyde de carbone est notamment comprise entre 1 et 300 bar et de préférence entre 10 et 100 bar.

La présente invention sera plus complètement décrite à l'aide des exemples suivants qui ne doivent pas être considérés comme limitatifs de l'invention.

Dans les exemples suivants les abréviations suivantes signifient :

$PCy_3$ = tricyclohexyl phosphine

$$TT = \text{taux de transformation} = \frac{\text{quantité de dérivé aromatique halogéné transformée}}{\text{quantité de dérivé aromatique halogéné introduite}} \times 100$$

$$RT = \text{rendement sur produit transformé} = \frac{\text{quantité de produit désiré formé (mol)}}{\text{quantité de produit transformé (mol)}} \times 100$$

$$RR = \text{rendement sur produit introduit} = \frac{\text{quantité de produit désiré formé (mol)}}{\text{quantité de dérivé aromatique halogené introduite (mol)}}$$

EXEMPLE 1 :

Dans un autoclave en Hastelloy HB2 on introduit :

| | |
|---|---|
| - Chloro benzène | 50 mmol |
| - $Pd(OA_o)_2$ | 1 mmol |
| - $NEt_3$ | 110 mmol |
| - $PCy_3$ | 5 mmol |
| - $H_2O$ | 15 ml |
| - Toluène | 10 ml |

On pressurise à 15 bar de CO et chauffe à 180° C pendant 5 h 20 mn.

On dégaze le réacteur. Après analyse en chromatographie liquide on obtient le résultat suivant : RR en $C_6H_5$ COOH = 88 %

EXEMPLE 2 :

Dans un autoclave en Hastelloy HB2 on introduit :

| | |
|---|---|
| - Chloro-4 méthoxybenzène | 50 mmol |
| - Tributylamine | 110 mmol |
| - $Pd(DBA)(PCy_3)_2$ | 1 mmol |
| - $PCy_3$ | 3 mmol |
| - $H_2O$ | 15 ml |
| - Toluène | 10 ml |

On pressurise à 15 bar de CO et chauffe à 180 °C pendant 22 h 40 mn.

Après traitement de la masse réactionnelle, on obtient un solide blanc dont l'analyse en RMN 1H confirme la formule attendue :
$CH_3O\ C_6H_4\ COOH$

EXEMPLE 3 :

Dans un autoclave en Hastelloy HB2 on introduit :

| | |
|---|---|
| - Trifluorométhyl-4 chlorobenzène | 50 mmol |
| - $NEt_3$ | 110 mmol |
| - $Pd(OAc)_2$ | 1 mmol |
| - $PCy_3$ | 5 mmol |
| - $H_2O$ | 15 ml |
| - Toluene | 10 ml |

On pressurise à 50 bar de CO et chauffe à 180 °C pendant 18 h.

On dégaze le réacteur. Après traitement de la masse réactionnelle, on obtient un solide blanc dont l'analyse en RMN 1H confirme la structure attendue :
$CF_3\ C_6H_4\ COOH$

EXEMPLE 4 :

Dans un autoclave en Hastelloy HB2 on introduit :

| | |
|---|---|
| - Fluoro-3 chloro benzène | 50 mmol |
| - $NEt_3$ | 5 mmol |
| - $Pd(OAc)_2$ | 1 mmol |
| - NaOH | 110 mmol |
| - $PCy_3$ | 5 mmol |
| - $H_2O$ | 15 ml |
| - Toluène | 10 ml |

On pressurise à 15 bar de CO et chauffe à 200 °C pendant 3 h 10 mn.

On dégaze le réacteur. Après traitement de la masse réactionnelle, on obtient un solide blanc dont l'analyse en RMN 1H confirme la structure attendue :
$F\ C_6H_4\ COOH$

## Revendications

1. Procédé d'hydroxycarbonylation de composés aromatiques halogénés caractérisé en ce qu'on met en présence un dérivé aromatique chloré, un catalyseur à base de palladium, une phosphine présentant un

Pka supérieur à 7 en présence d'une base avec de l'eau et de l'oxyde de carbone.

2. Procédé selon la revendication 1 caractérisé en ce que le dérivé chloroaromatique répond à la formule ArCl dans laquelle Ar représente un radical aromatique mono, polycyclique ou hétérocyclique éventuellement substitué.

3. Procédé selon la revendication 2 caractérisé en ce que Ar représente un radical benzénique, éventuellement substitué par un groupe alkyle, alkoxy, alkylcarbonyle, cycloalkyle, cycloalkoxy, alkylcarbonyloxy, cycloalkylcarbonyloxy, aryle, aryloxy, arylcarbonyloxy, aryloxycarbonyle, fluoro, chloro, halogénoalkyle, halogénoalkoxy, halogénocycloalkyle, halogénocycloalkyloxy, halogénoaryle, halogénoaryloxy, les motifs alkyle ou alkoxy ayant de 1 à 12 atomes de carbone.

4. Procédé selon la revendication 3 caractérisé en ce que le composé ArCl est le chlorobenzène, le chlorométhoxybenzène, le trifluorométhylbenzène.

5. Procédé selon la revendication 1 caractérisé en ce que la phosphine est choisie parmi les phosphines présentant un angle de cône compris entre 160 et 180°.

6. Procédé selon les revendications 1 et 5, caractérisé en ce que le phosphine est choisie parmi la tricyclohexylphosphine, la triisopropylphosphine, la dicyclohexylphénylphosphine.

7. Procédé selon les revendications 5 et 6, caractérisé en ce que la phosphine est la tricyclohexylphosphine.

8. Procédé selon la revendication 1 caractérisé en ce qu'on ajoute une base choisie parmi les amines tertiaires et les bases minérales en quantité molaire supérieure au dérivé aromatique.

9. Procédé selon la revendication 1 caractérisé en ce que la réaction a lieu dans un excès de réactif ou en présence d'un solvant choisi parmi les dérivés aromatiques ou aliphatiques hydrocarbonés, éventuellement halogénés, les éthers, les amides, les nitriles.

10. Procédé selon la revendication 1 caractérisé en ce que la quantité de palladium exprimé en milliatome-gramme de métal noble ou en millimoles de dérivé métallique par litre est comprise dans l'intervalle $10^{-5}$ à 100.

11. Procédé selon les revendications 1 et 10 caractérisé en ce que la quantité de phosphine est telle que le nombre des atomes-grammes de phosphore au nombre des atomes-grammes de métal soit compris dans l'intervalle 2 à 10000.

12. Procédé selon la revendication 1 caractérisé en ce que la pression réactionnelle est comprise entre 1 et 300 bar et de préférence entre 10 et 100 bar.

13. Procédé selon la revendication 1, caractérisé en ce que la température réactionnelle est comprise entre 50 et 250°C et de préférence comprise entre 100 et 200°C.

14. Procédé de mise en oeuvre caractérisé en ce qu'on introduit dans un solvant un complexe du palladium de formule (I) suivante :

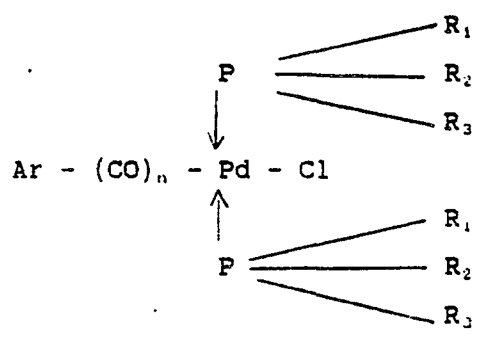

(I)

dans laquelle :

. $R_1$, $R_2$, $R_3$ représentent chacun des groupes identiques ou différents choisis parmi les radicaux cyclohexyle, isopropyle, l'un des groupes $R_1$, $R_2$ ou $R_3$ pouvant être remplacé par un groupe phényle lorsque les deux autres représentent un groupe cyclohexyle,

. Ar représente un radical aromatique mono, polycyclique ou hétérocyclique

. n est égal à 0 ou 1 avec un dérivé chloroaromatique , du monoxyde de carbone et de l'eau en présence éventuellement d'un excès de phosphine.

15. Procédé de mise en oeuvre caractérisé en ce qu'on introduit dans un solvant un complexe du palladium de formule (II) suivante :

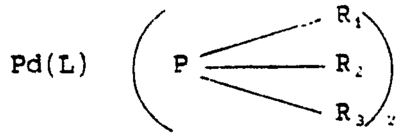

(II)

dans laquelle :

. $R_1$, $R_2$, $R_3$ ont la même signification que dans la formule (I)

. L représente la dibenzylidène acétone ou un groupe alkylène avec un dérivé chloroaromatique, du monoxyde de carbone et de l'eau en présence éventuellement d'un excès de phosphine.

16. Procédé de mise en oeuvre caractérisé en ce qu'on introduit dans un solvant un complexe de palladium de formule $Pd(L)_3$ dans laquelle L a la même signification que dans la formule (II), un dérivé chloroaromatique, du monoxyde de carbone et de l'eau en présence d'une phosphine de formule :

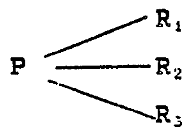

dans laquelle :

$R_1$, $R_2$, $R_3$ ayant la même signification que dans la formule (I).

17. Procédé de mise en oeuvre caractérisé en ce qu'on introduit dans un solvant un complexe de palladium à l'état d'oxydation II, un dérivé chloroaromatique, du monoxyde de carbone et de l'eau en présence d'une phosphine de formule

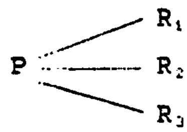

dans laquelle :

$R_1$, $R_2$, $R_3$ ayant la même signification que dans la formule (I).

18. Procédé selon la revendication 17 caractérisé en ce que le complexe de palladium à l'état d'oxydation II est choisi parmi le dichlorure, le dibromure, le diiodure de palladium, le diacétate de palladium, le sulfate de palladium, l'oxyde de palladium.

Office européen des brevets

# RAPPORT DE RECHERCHE EUROPEENNE

Numero de la demande

EP 89 40 2644

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X<br><br>Y | EP-A-0 283 194 (NIHON NOHYAKU)<br>* revendications 1-12 *<br>* revendications 1-3 * | 1-4<br><br>1,6 | C 07 C 51/10<br>C 07 C 63/06<br>C 07 C 65/21<br>C 07 C 63/70 |
| Y | EP-A-0 255 794 (RHONE-POULENC)<br>* revendications 1-3; page 3, lignes 21-26 * | 1,6 | |
| A | EP-A-0 273 553 (STAUFFER)<br>* revendications * | 1 | |
| A | PATENT ABSTRACTS OF JAPAN<br>vol. 12, no. 40 (C-474)(2887), 5 février 1988; & JP - A - 62 190145 (ASAHI) 20.08.1987 | 1 | |
| A | US-A-2 565 461 (H. BLISS et al.)<br>* revendications * | 1 | |
| D,A | BEILSTEINS HANDBUCH DER ORGANISCHEN CHEMIE<br>4ème édition, tome 9, II supplément, Springer-Verlag, Berlin, 1949 * page 72, 2ème paragraphe, lignes 3,4 * | 1 | **DOMAINES TECHNIQUES RECHERCHES (Int. Cl.5)** |
| D,A | JOURNAL OF THE AMERICAN CHEMICAL SOCIETY<br>tome 82, no. 19, 7 octobre 1960, pages 5791-5794, American Chemical Society, Washington, D.C., USA; WM. A. HENDERSON et al.: "The Basicity of Phosphines" * page 5792, table 1 *<br>-/- | 1 | C 07 C 51/00<br>C 07 C 63/00<br>C 07 C 65/00 |

Le présent rapport a été établi pour toutes les revendications

| Lieu de la recherche | Date d'achèvement de la recherche | Examinateur |
|---|---|---|
| BERLIN | 15-12-1989 | PROBERT C.L. |

**CATEGORIE DES DOCUMENTS CITES**

X : *particulièrement pertinent à lui seul*
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 89 40 2644

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | JOURNAL OF THE AMERICAN CHEMICAL SOCIETY vol. 92, no. 7, 8 avril 1970, pages 2956-2965, American Chemical Society, Washington, D.C., USA; C.A. TOLMAN: "Phosphorous Ligand Exchange Equilibra on Zerovalent Nickel. A Dominant Role for Steric Effects" * page 2963, table IV; page 2964, table VI * | 5 | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| BERLIN | 15-12-1989 | PROBERT C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant